# NOUVEAU FASCICULE DE BREVET EUROPEEN

(11) **EP 1 800 660 B2**
(45) Date de publication et mention de la décision concernant l'opposition: **27.03.2019**
(45) Mention de la délivrance du brevet: 17.02.2016
(21) Numéro de dépôt: 06291982.4
(22) Date de dépôt: 19.12.2006
(51) Int. Cl.: A61K 8/81, A61K 8/89, A61Q 5/06, A61Q 19/00, A61K 8/891, A61K 8/892, A61K 8/895, A61K 8/898

(54) **Composition cosmétique comprenant au moins un poly(vinyllactame) cationique, au moins un polymère cationique réticulé particulier et au moins une silicone non volatile**
Kosmetische Zusammensetzung, die mindestens ein kationisches Poly(vinyllactam), mindestens ein besonders vernetztes kationisches Polymer und mindestens ein nicht flüchtiges Silikon enthält
Cosmetic ingredient comprising at least one cationic poly(vinyllactam), at least one specific cross-linked cationic polymer and at least one non-volatile silicon

(30) Priorité: 22.12.2005 FR 0513191
(43) Date de publication de la demande: 27.06.2007
(73) Titulaire: L'Oréal, 75008 Paris (FR)
(72) Inventeur: Pasquet, Dorothée, 92270 Bois-Colombes (FR)
(74) Mandataire: Casalonga, Axel

(56) Documents cités:
- EP-A2- 1 319 390
- WO-A-00/68282
- WO-A-02/058646
- WO-A1-00/49999
- FR-A1- 2 831 800
- FR-A1- 2 845 908

## Description

La présente invention concerne une composition cosmétique pour le traitement des fibres kératiniques, en particulier des fibres kératiniques humaines telles que les cheveux, comprenant au moins un polymère poly(vinyllactame) cationique, au moins un polymère cationique réticulé particulier et au moins une silicone non volatile, une utilisation de cette composition pour la mise en forme et/ou le maintien de la coiffure ainsi qu'un procédé de traitement cosmétique la mettant en oeuvre.

Dans le domaine du coiffage, en particulier parmi les produits capillaires destinés à la mise en forme et/ou au maintien de la coiffure, les compositions capillaires sont généralement constituées d'une solution le plus souvent alcoolique ou aqueuse et d'un ou de plusieurs polymères fixants dont la fonction est de former un film à la surface des fibres kératiniques à fixer afin de réaliser des soudures entre celles-ci.

Ces compositions peuvent se présenter sous la forme de gels, de lotions capillaires qui sont généralement appliquées sur des cheveux mouillés avant d'effectuer un brushing ou un séchage.

Ces compositions peuvent être également conditionnées soit dans un récipient aérosol approprié mis sous pression à l'aide d'un agent propulseur, soit dans un flacon pompe.

Les polymères fixants classiquement utilisés dans ces compositions capillaires sont le plus souvent des polymères fixants anioniques ou cationiques et sont généralement formulés en mélange avec divers adjuvants cosmétiques.

Bien que les polymères fixants anioniques classiquement utilisés dans ces compositions capillaires confèrent généralement de bonnes propriétés fixantes, ces derniers ne permettent pas d'obtenir des propriétés cosmétiques satisfaisantes, en particulier sur des cheveux humides ou mouillés.

En effet, les cheveux ainsi traités avec de tels polymères fixants anioniques deviennent généralement rêches, difficiles à démêler et présentent un toucher et un aspect visuel désagréable.

En outre, les polymères fixants cationiques classiquement utilisés dans des compositions capillaires confèrent généralement de bonnes propriétés cosmétiques mais ne permettent pas d'obtenir un maintien et/ou une fixation de la coiffure satisfaisant.

Le document WO 02/058646 concerne des composition de traitement des matières kératiniques, en particulier des cheveux, comprenant un agent protecteur ou conditionneur ainsi qu'un moins un poly(alkyl)vinyllactame cationique. Ces associations permettent d'améliorer les dépôts de l'agent protecteur ou conditionneur des matières kératiniques ainsi que les propriétés cosmétiques.

Par ailleurs il est connu que les polymères de type Salcare SC96 sont des polymères fixants (extrait du CTFA édition 2004, volume 2 à la page 1451).

Cependant, il n'est pas connu que ces polymères sont capables de conférer de bonnes propriétés cosmétiques.

Il existe donc un réel besoin de trouver des compositions cosmétiques, notamment pour le coiffage, qui ne présentent pas l'ensemble des inconvénients décrits ci-dessus, et qui en particulier confèrent à la chevelure un niveau élevé de fixation pour obtenir une mise en forme et/ou un maintien de la coiffure satisfaisant tout en procurant de bonnes propriétés cosmétiques, notamment en termes de douceur, de démêlage, d'aspect et de toucher.

De manière surprenante et avantageuse, la Demanderesse vient de découvrir qu'en associant un polymère poly(vinyllactame) cationique, un polymère cationique réticulé particulier et une silicone non volatile, il était possible notamment d'obtenir à la fois de bonnes propriétés fixantes et de bonnes propriétés cosmétiques, en particulier en termes de douceur, de démêlage, d'aspect et de toucher.

La présente invention a donc notamment pour objet une composition cosmétique pour le traitement des fibres kératiniques, en particulier des fibres kératiniques humaines telles que les cheveux, comprenant une telle association.

Un autre objet de la présente invention consiste en une utilisation de la composition cosmétique selon l'invention pour la mise en forme et/ou le maintien de la coiffure.

L'invention a encore pour objet un dispositif aérosol comprenant la composition cosmétique selon l'invention.

Selon l'invention, la composition cosmétique pour le traitement des fibres kératiniques, en particulier des fibres kératiniques humaines telles que les cheveux, comprend, dans un milieu cosmétiquement acceptable,
- au moins un polymère poly(vinyllactame) cationique comprenant :
   - a) au moins un monomère de type vinyl lactame ou alkylvinyllactame;
   - b) au moins un monomère de structures (Ia) ou (Ib) suivantes : 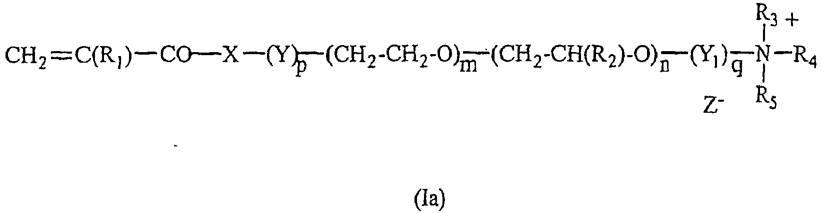 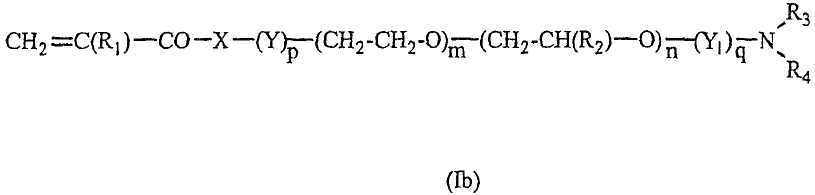 dans lesquelles :
      X désigne un atome d'oxygène ou un radical NR₆,
      R₁ et R₆ désignent, indépendamment l'un de l'autre, un atome d'hydrogène ou un radical alkyl linéaire ou ramifié en C₁-C₅,
      R₂ désigne un radical alkyle linéaire ou ramifié en C₁-C₄,
      R₃, R₄ et R₅ désignent, indépendamment l'un de l'autre, un atome d'hydrogène, un radical alkyle linéaire ou ramifié en C₁-C₃₀ ou un radical de formule (II) : 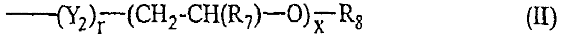
      Y, Y₁ et Y₂ désignent, indépendamment l'un de l'autre, un radical alkylène linéaire ou ramifié en C₂-C₁₆,
      R₇ désigne un atome d'hydrogène, ou un radical alkyle linéaire ou ramifié en C₁-C₄ ou un radical hydroxyalkyle linéaire ou ramifié en C₁-C₄,
      R₈ désigne un atome d'hydrogène ou un radical alkyle linéaire ou ramifié en C₁-C₃₀,
      p, q et r désignent, indépendamment l'un de l'autre, soit la valeur zéro, soit la valeur 1,
      m et n désignent, indépendamment l'un de l'autre, un nombre entier allant de 0 à 100,
      x désigne un nombre entier allant de 1 à 100,
      Z désigne un anion d'acide organique ou minéral,
      sous réserve que :
         - l'un au moins des substituants R₃, R₄, R₅ ou R₈ désigne un radical alkyle linéaire ou ramifié en C₉-C₃₀,
         - si m ou n est différent de zéro, alors q est égal à 1,
         - si m ou n sont égaux à zéro, alors p ou q est égal à 0 ;
- au moins un polymère cationique réticulé choisi parmi les homo ou copolymères de chlorure de méthacryloyloxyalkyl(C₁-C₄)trialkyl(C₁-C₄) ammonium ; et
- au moins une silicone non volatile, en une quantité variant de 0,01 à 10% en poids, par rapport au poids total de la composition cosmétique.

Les polymères poly(vinyllactames) cationiques utilisés dans la composition cosmétique selon l'invention comprennent :
- a) au moins un monomère de type vinyl lactame ou alkylvinyllactame;
- b) au moins un monomère de structures (Ia) ou (Ib) suivantes : 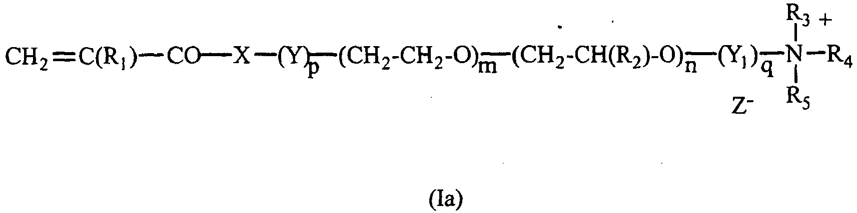 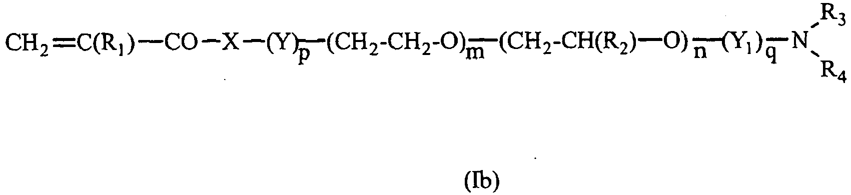 dans lesquelles :
   X désigne un atome d'oxygène ou un radical NR₆,
   R₁ et R₆ désignent, indépendamment l'un de l'autre, un atome d'hydrogène ou un radical alkyl linéaire ou ramifié en C₁-C₅,
   R₂ désigne un radical alkyle linéaire ou ramifié en C₁-C₄,
   R₃, R₄ et R₅ désignent, indépendamment l'un de l'autre, un atome d'hydrogène, un radical alkyle linéaire ou ramifié en C₁-C₃₀ ou un radical de formule (II) : 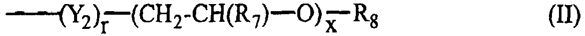
      Y, Y₁ et Y₂ désignent, indépendamment l'un de l'autre, un radical alkylène linéaire ou ramifié en C₂-C₁₆,
      R₇ désigne un atome d'hydrogène, ou un radical alkyle linéaire ou ramifié en C₁-C₄ ou un radical hydroxyalkyle linéaire ou ramifié en C₁-C₄,
      R₈ désigne un atome d'hydrogène ou un radical alkyle linéaire ou ramifié en C₁-C₃₀,
      p, q et r désignent, indépendamment l'un de l'autre, soit la valeur zéro, soit la valeur 1,
      m et n désignent, indépendamment l'un de l'autre, un nombre entier allant de 0 à 100,
      x désigne un nombre entier allant de 1 à 100,
      Z désigne un anion d'acide organique ou minéral,
      sous réserve que :
         - l'un au moins des substituants R₃, R₄, R₅ ou R₈ désigne un radical alkyle linéaire ou ramifié en C₉-C₃₀,
         - si m ou n est différent de zéro, alors q est égal à 1,
         - si m ou n sont égaux à zéro, alors p ou q est égal à 0 ;

Les polymères poly(vinyllactames) cationiques utilisés dans la composition cosmétique selon l'invention peuvent être réticulés ou non et peuvent aussi être des polymères blocs.

De préférence, le contre ion Z⁻ des monomères de formule (Ia) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (Ia) pour laquelle, encore plus préférentiellement, m et n sont égaux à zéro.

Le monomère vinyl lactame ou alkylvinyllactame est de préférence un composé de structure (III) : 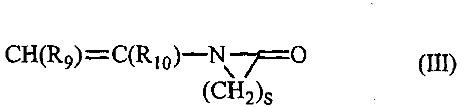 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (III) est la vinylpyrrolidone.

Les polymères poly(vinyllactame) cationiques utilisés dans la composition selon l'invention peuvent également contenir un ou plusieurs monomères supplémentaires, de préférence cationiques ou non ioniques.

A titre de composés utilisés de manière plus particulièrement préférée selon l'invention, on peut citer les terpolymères suivants comprenant au moins :
a)-un monomère de formule (III),
b)-un monomère de formule (Ia) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (Ib) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95% de monomère (a), 0,1 à 55% de monomère (c) et 0,25 à 50% de monomère (b).

De tels polymères sont décrits dans la demande de brevet WO-00/68282 dont le contenu fait partie intégrante de l'invention.

Comme polymères poly(vinyllactame) cationiques selon l'invention, on utilise notamment les terpolymères vinylpyrrolidone / diméthylaminopropylméthacrylamide / tosylate de dodécyldiméthylméthacrylamidopropylammonium, les terpolymères vinylpyrrolidone /diméthylaminopropylméthacrylamide/ tosylate de cocoyldiméthyl-méthacrylamidopropylammonium, les terpolymères vinylpyrrolidone / diméthylaminopropylméthacrylamide / tosylate ou chlorure de lauryldiméthylméthacrylamidopropylammonium.

La masse moléculaire en poids des polymères poly(vinyllactame) cationiques utilisés dans la composition cosmétique selon la présente invention est de préférence comprise entre 500 et 20 000 000. Elle est plus particulièrement comprise entre 200 000 et 2 000 000 et encore plus préférentiellement comprise entre 400 000 et 800 000.

Un polymère particulièrement préféré est le polymère commercialisé sous la dénomination «Styleze W20» par la société ISP qui est un terpolymère de vinylpyrrolidone / de diméthylaminopropylméthacrylamide et de chlorure de lauryldiméthylméthacrylamidopropylammonium.

Le(s) polymère(s) poly(vinyllactame) cationiques(s) est ou sont présents dans la composition cosmétique selon l'invention en une quantité variant de 0,05 à 30% en poids, de préférence en une quantité variant de 0,1 à 15 % en poids et encore plus préférentiellement en une quantité variant de 0,2 à 10 % en poids, par rapport au poids total de la composition.

Au sens de la présente invention, on entend par « polymère cationique », tout polymère comprenant des groupements cationiques et/ou des groupements ionisables en groupements cationiques.

Les polymères réticulés cationiques choisis parmi les homo ou copolymères de chlorure de méthacryloyloxyalkyl(C₁-C₄)trialkyl(C₁-C₄)ammonium utilisés dans la composition cosmétique selon l'invention sont, de préférence, des polymères réticulés cationiques de chlorure de méthacryloyloxyéthyl triméthylammonium de type homopolymère ou copolymère avec l'acrylamide.

Les polymères réticulés de chlorure de méthacryloyloxyéthyl triméthylammonium utilisés dans la composition cosmétique selon l'invention, sont plus particulièrement choisis parmi les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide.

On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS.

On peut également utiliser avantageusement un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

Le(s) polymère(s) réticulé(s) cationiques(s) choisi(s) parmi les homo ou copolymères de chlorure de méthacryloyloxyalkyl(C₁-C₄)triakyl(C₁-C₄)ammonium est ou sont présents dans la composition cosmétique selon l'invention en une quantité variant de 0,01 à 30 % en poids, de préférence en une quantité variant de 0,05 à 20 % en poids et encore plus préférentiellement en une quantité variant de 0,1 à 10 % en poids, par rapport au poids total de la composition.

Dans tout ce qui précède, on entend désigner par silicone ou polysiloxane, en conformité avec l'acceptation générale, tout polymère ou oligomère organosilicié à structure linéaire, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxy ou acyloxyalkyls, des groupements ammonium quaternaire, des groupements anioniques tels que carboxylates, thioglycolates, sulfosuccinates, thiosulfates, phosphates et sulfates, cette liste n'étant bien entendu nullement limitative (silicones dites "organomodifiées").

Par silicone non volatile, on entend au sens de la présente invention, toute silicone dont le nombre d'atomes de silicium est supérieure à 7.

Les silicones non volatiles utilisées dans la composition cosmétique conformément à l'invention sont en particulier des polyorganosiloxanes insolubles dans la composition. Elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

On utilise des silicones de préférence des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM-445 Appendice C.

Parmi ces polyalkylsiloxanes, on peut citer à titre non limitatif les produits commerciaux suivants :
- les huiles SILBIONE® des séries 47 et 70 047 ou les huiles MIRASIL DM commercialisées par RHODIA CHIMIE telles que par exemple l'huile 70 047 V 500 000, ou l'huile MIRASIL DM 300 000 ;
- les huiles de la série MIRASIL® DM commercialisées par la société RHODIA CHIMIE ;
- les huiles de la série 200 de la société DOW CORNING telles que plus particulièrement la DC200 de viscosité 60 000 Cst;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL® WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE® de la série 70 641 de RHODIA CHIMIE ;
- les huiles des séries RHODORSIL 70 633 et 763 de RHODIA CHIMIE ;
- l'huile DOW CORNING 556® COSMETIC GRAD FLUID de DOW CORNING ;
- les silicones de la série PK de BAYER comme le produit PK20;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des poids moléculaires moyens en poids élevés compris entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C12) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous la dénomination Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (IV) : 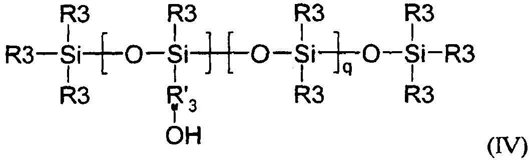 dans laquelle les radicaux R₃ identiques ou différentes sont choisis parmi les radicaux méthyle et phényle ; au moins 60 % en mole des radicaux R₃ désignant méthyle ; le radical R'₃ est un chaînon alkylène divalent hydrocarboné en C₂-C₁₈ ; p est compris entre 1 et 30 inclus ; q est compris entre 1 et 150 inclus ;
- des groupements acyloxyalkyle comme par exemple les polyorganosiloxanes décrits dans le brevet US-A-4957732 et répondant à la formule (V) : 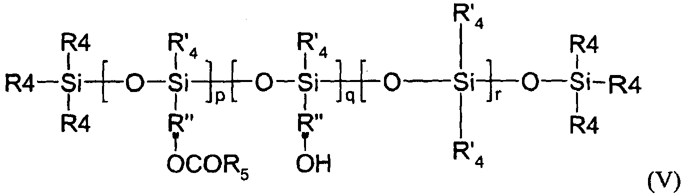 dans laquelle :
   R₄ désigne un groupement méthyle, phényl, -OCOR₅, hydroxyle, un seul des radicaux R₄ par atome de silicium pouvant être OH ;
   R'₄ désigne méthyle, phényle ; au moins 60 % en proportion molaire de l'ensemble des radicaux R₄ et R'₄ désignant méthyle ;
   R₅ désigne alkyle ou alcényle en C₈-C₂₀ ;
   R" désigne un radical alkylène hydrocarboné divalent, linéaire ou ramifié en C₂-C₁₈ ;
   r est compris entre 1 et 120 inclus ;
   p est compris entre 1 et 30 ;
   q est égal à 0 ou est inférieur à 0,5 p, p+q étant compris entre 1 et 30 ; les polyorganosiloxanes de formule (VI) peuvent contenir des groupements : 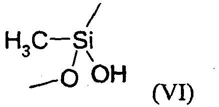
   dans des proportions ne dépassant pas 15 % de la somme p+q+r.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur ladite chaîne principale. Ces polymères sont par exemple décrits dans les demandes de brevet EP-A-412 704, EP-A-412 707, EP-A-640 105 et WO 95/00578, EP-A-582152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 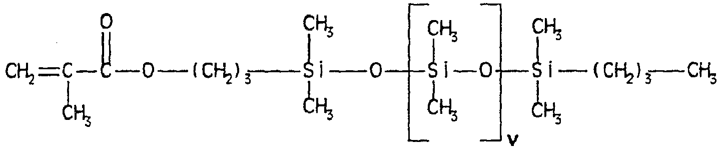
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles polydiméthylsiloxane ayant une viscosité comprise entre 0,2 et 2,5 m²/s à 25° C telles que les huiles de la série DC200 de DOW CORNING en particulier celle de viscosité 60 000 Cst, des séries SILBIONE 70047 et 47 et plus particulièrement l'huile 70 047 V 500 000 commercialisées par la société RHODIA CHIMIE, les polyalkylsiloxanes à groupements terminaux diméthylsilanol ou les polyalkylarylsiloxanes tels que l'huile SILBIONE 70641 V 200 commercialisée par la société RHODIA CHIMIE ;
- la résine d'organopolysiloxane commercialisée sous la dénomination DOW CORNING 593 ;
- les polysiloxanes à groupements aminés tels que les amodiméthicones ou les triméthylsilylamodiméthicone ;
- les mélanges de deux polydiméthylsiloxanes constituées d'une gomme et d'une huile de viscosités différentes ;
- les mélanges d'organosiloxanes et de silicones cycliques.

Le(s) silicone(s) non volatile(s) utilisée(s) dans la composition cosmétique selon l'invention est ou sont présentes en une quantité variant de 0,01 à 10% en poids, de préférence en une quantité variant de 0,05 à 10% en poids, et encore plus préférentiellement en une quantité variant de 0,1 à 10% en poids, par rapport au poids total de la composition cosmétique.

De préférence, le rapport pondéral polyvinyllactame cationique de l'invention / polymère cationique réticulé de l'invention est compris entre 0,1 et 10, le rapport pondéral poly(vinyllactame) cationique de l'invention / silicone non volatile de l'invention est compris entre 0,1 et 10 et le rapport pondéral polymère cationique réticulé de l'invention / silicone non volatile de l'invention est compris entre 0,2 et 5.

La composition cosmétique selon l'invention peut comprendre en outre un ou plusieurs polymères fixants différents des polymères de l'invention.

Par polymère fixant, on entend au sens de la présente invention tout polymère permettant de conférer une forme ou de maintenir une forme ou une coiffure donnée.

Les polymères fixants utilisables dans la composition cosmétique sont notamment choisis parmi les polymères cationiques, anioniques, amphotères, non ioniques et leurs mélanges.

Les polymères fixants cationiques utilisables dans la composition cosmétique sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 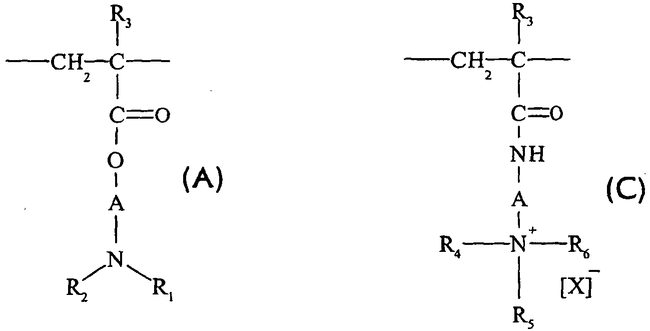 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques ou méthacryliques ou de leurs esters, de vinyllactames tels que la vinylpyrrolidone ou le vinylcaprolactame, d'esters vinyliques.
   Ainsi parmi ces copolymères de la famille (1), on peut citer :
   - les terpolymères méthacrylate de diméthylaminoéthyle/ vinylcaprolactame/vinylpyrrolidone tels que le produit commercialisé sous la dénomination GAFFIX^{®} VC 713 par la société ISP, et
   - les copolymères vinylpyrrolidone/méthacrylamide de diméthylaminopropyle quaternisé tels que notamment le produit commercialisé sous la dénomination "GAFQUAT^{®} HS 100" par la société ISP ;
(2) les polysaccharides cationiques, de préférence à ammonium quaternaire, tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL;
(3) les copolymères quaternaires de vinylpyrrolidone et de vinylimidazole ;
(4) les chitosanes ou leurs sels ; les sels utilisables sont en particulier l'acétate, le lactate, le glutamate, le gluconate ou le pyrrolidone-carboxylate de chitosane.
   Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER^{®} PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 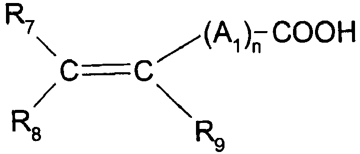 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID, et ULTRAHOLD^{®} par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371 ou proposés sous la dénomination QUADRAMER par la Société AMERICAN CYANAMID. On peut également citer les terpolymères acide méthacrylique/acrylate d'éthyle/acrylate de tertiobutyle tels que le produit commercialisé sous la dénomination LUVIMER^{®} 100 P par la société BASF.
   On peut aussi citer les copolymères acide méthacrylique/acide acrylique/acrylate d'éthyle/méthacrylate de méthyle en dispersion aqueuse, commercialisé sous la dénomination AMERHOLD^{®} DR 25 par la société AMERCHOL.
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-acyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ^{®} AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
Ces polymères sont par exemple décrits dans les brevets français n^{os} 2 350 384 et 2 357 241 de la demanderesse. E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations Flexan^{®} 500 et Flexan^{®} 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par Henkel.

De préférence, les polymères fixants anioniques sont choisis parmi les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX^{®} A par la société BASF.

Parmi les polymères fixants anioniques cités ci-dessus, on préfère plus particulièrement utiliser dans le cadre de la présente invention les copolymères méthylvinyléther/anhydride maléique monoestérifiés vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkyl-méthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.

Les comonomères basiques préférés sont des méthacrylates d'aminoéthyle, de butylaminoéthyle, de N,N'-diméthylaminoéthyle, de N-tertio-butylaminoéthyle.

On utilise particulièrement les copolymères dont la dénomination CTFA (4ème Ed., 1991) est octylacrylamide/acrylates/ butylaminoethyl methacrylate copolymer, tels que les produits vendus sous la dénomination AMPHOMER^{®} ou LOVOCRYL^{®} 47 par la société NATIONAL STARCH.
(3) les polyaminoamides réticulés et acylés partiellement ou totalement dérivant de polyaminoamides de formule générale : 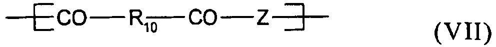 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 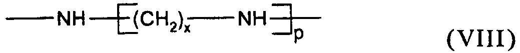 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (VIII) ci-dessus, dans lequel x=2 et p=1 et qui dérive de l'éthylène-diamine, ou le groupe dérivant de la pipérazine : 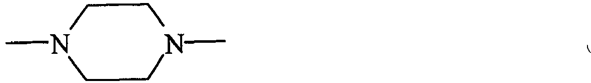
   c) dans les proportions de 0 à 20 % en moles, le groupe -NH-(CH₂)₆-NH- dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.

Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 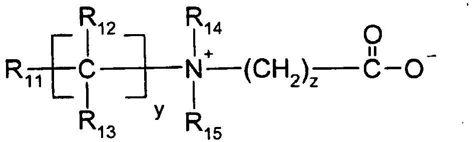 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.

Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.

A titre d'exemple, on peut citer les copolymères méthacrylate de méthyle/diméthyl-carboxyméthylammonioéthylméthacrylate de méthyle, tels que le produit vendu sous la dénomination DIAFORMER Z301 par la société SANDOZ.
(5) les polymères dérivés du chitosane comportant des motifs monomères répondant aux formules suivantes : 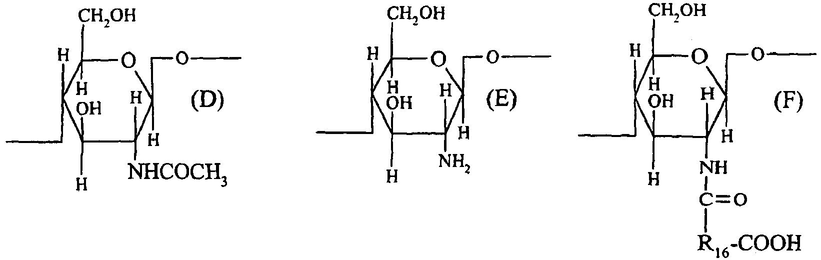 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 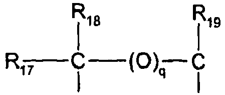
   dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères contenant des motifs répondant à la formule générale (IX) sont, par exemple, décrits dans le brevet français 1 400 366 : 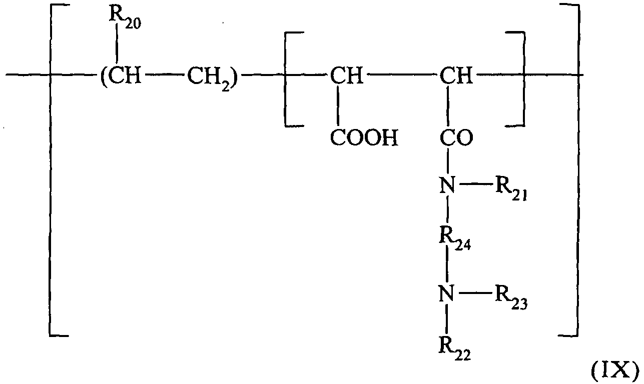 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendu sous la dénomination "EVALSAN" par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (X)

      où D désigne un groupe 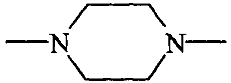 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (XI')

      où D désigne un groupe 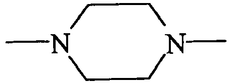 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères décrits ci-dessus, les plus particulièrement préférés selon l'invention sont ceux de la famille (3) tels que les copolymères dont la dénomination CTFA est Octylacrylamide/ acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthyl-carboxyméthylammonio-éthyl-méthacrylate de méthyle vendu par exemple sous la dénomination DIAFORMER^{®} Z301 par la société SANDOZ.

Les polymères fixants non ioniques utilisables dans la composition sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol^{®} PLUS par la société BASF ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.

Les groupes alkyle des polymères non ioniques mentionnés ci-dessus ont, de préférence, de 1 à 6 atomes de carbone.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR^{®} et LUVISET^{®} Si PUR par la société BASF.

Le ou les polymères fixants additionnel(s) est ou sont présents dans la composition cosmétique selon l'invention en une quantité variant de 0,01 à 20 % en poids, de préférence de 0,05 à 15 % en poids, et encore plus préférentiellement de 0,1 à 10 % en poids, par rapport au poids total de la composition cosmétique.

La composition cosmétique selon l'invention peut en outre comprendre un ou plusieurs polymères épaississants encore appelé(s) "agents d'ajustement de la rhéologie".

Les agents d'ajustement de la rhéologie peuvent être choisis parmi les amides d'acides gras (diéthanol- ou monoéthanol-amide de coprah, monoéthanolamide d'acide alkyl éther carboxylique oxyéthyléné), les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs tels que décrits ci-dessous autres que les polymères de l'invention.

Les polymères associatifs utilisables dans la composition cosmétique sont des polymères hydrosolubles capables, dans un milieu aqueux, de s'associer réversiblement entre eux ou avec d'autres molécules.

Leur structure chimique comprend des zones hydrophiles, et des zones hydrophobes caractérisées par au moins une chaîne grasse.

Les polymères associatifs utilisables dans la composition cosmétique peuvent être de type anionique, cationique, amphotère et de préférence non ionique.

Parmi les polymères associatifs de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (XV) suivante :

   CH₂ = CR'CH₂OBₙR (XV)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (XV) plus particulièrement préféré est un motif dans lequel R' désigne H, n est égal à 10, et R désigne un radical stéaryl (C₁₈).

Des polymères associatifs anioniques de ce type sont décrits et préparés, selon un procédé de polymérisation en émulsion, dans le brevet EP-0 216 479.

Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (XV), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC80® et SALCARE SC90® qui sont des émulsions aqueuses à 30% d'un terpolymère réticulé d'acide méthacrylique, d'acrylate d'éthyle et de steareth-10-allyl éther (40/50/10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.

De préférence, ces polymères sont choisis parmi ceux dont le motif hydrophile de type acide carboxylique insaturé oléfinique correspond au monomère de formule (XVI) suivante : 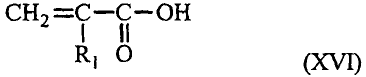 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (XVII) suivante 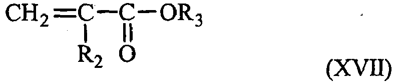 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4 509 949.

Parmi ce type de polymères associatifs anioniques, on utilisera plus particulièrement des polymères formés à partir d'un mélange de monomères comprenant :
(i) essentiellement de l'acide acrylique,
(ii) un ester de formule (XVII) décrite ci-dessus et dans laquelle R₂ désigne H ou CH₃, R₃ désignant un radical alkyle ayant de 12 à 22 atomes de carbone, et
(iii) un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.

Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.
- (III) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608® par la société NEWPHASE TECHNOLOGIES.
- (IV) les terpolymères acryliques comprenant :
   (a) environ 20% à 70% en poids d'un acide carboxylique à insaturation α,β-monoéthyténique,
   (b) environ 20 à 80% en poids d'un monomère à insaturation α,β-monoéthyténique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (400E) en dispersion aqueuse à 25%.
- (V) les copolymères comportant parmi leurs monomères un acide carboxylique à insaturation α,β-monoéthyténique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

Parmi les polymères associatifs de type cationique, on peut citer:
- (I) les polyuréthanes associatifs cationiques dont la famille a été décrite par la demanderesse dans la demande de brevet français N° 0009609; elle peut être représentée par la formule générale (XVIII) suivante :

   R-X-(P)n-[L-(Y)m]r-L'-(P')p-X'-R' (XVIII)

   dans laquelle :
   R et R', identiques ou différents, représentent un groupement hydrophobe ou un atome d'hydrogène ;
   X et X', identiques ou différents, représentent un groupement comportant une fonction amine portant ou non un groupement hydrophobe, ou encore le groupement L" ;
   L, L' et L", identiques ou différents, représentent un groupement dérivé d'un diisocyanate ;
   P et P', identiques ou différents, représentent un groupement comportant une fonction amine portant ou non un groupement hydrophobe ;
   Y représente un groupement hydrophile ;
   r est un nombre entier compris entre 1 et 100, de préférence entre 1 et 50 et en particulier entre 1 et 25, n, m, et p valent chacun indépendamment des autres entre 0 et 1000 ;
   la molécule contenant au moins une fonction amine protonée ou quaternisée et au moins un groupement hydrophobe.

Dans un mode de réalisation préféré de ces polyuréthanes, les seuls groupements hydrophobes sont les groupes R et R' aux extrémités de chaîne.

Une famille préférée de polyuréthanes associatifs cationiques est celle correspondant à la formule (XVIII) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (XVIII) ci-dessus dans laquelle :
R et R' représentent tous les deux indépendamment un groupement hydrophobe, X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (XVIII) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 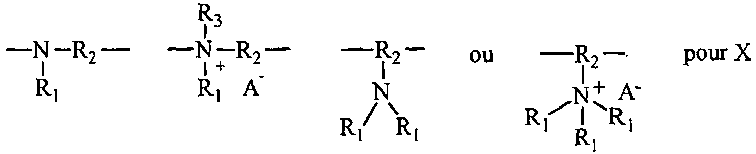 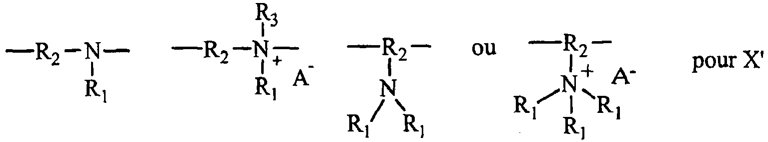 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 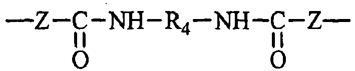 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 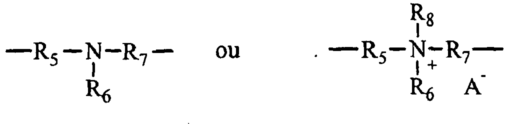 ou 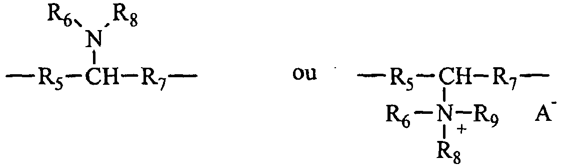 ou 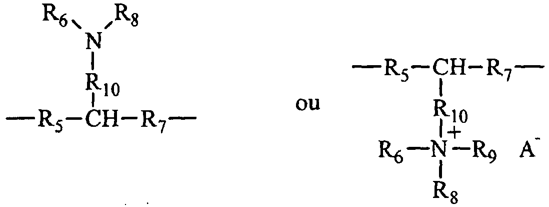 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A⁻ est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (XVIII) utilisables selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" utilisable selon la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (XVIII) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)n-ZH,

ou

HZ-(P')p-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (XVIII) est un diisocyanate correspondant à la formule :

O=C=N-R₄-N=C=O

dans laquelle R₄ est défini plus haut.

A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (XVIII) est un composé hydrophobe destiné à former les groupes hydrophobes terminaux du polymère de formule (XVIII).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné alpha-hydroxyle.

Le groupe hydrophobe du polyuréthane de formule (XVIII) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (XVIII) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.
- (II) les dérivés de cellulose quaternisée et les polyacrylates à groupements latéraux aminés non cycliques.

Les dérivés de cellulose quaternisée sont en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C12) et CRODACEL QS® (alkyle en C₁₈) commercialisés par la société CRODA.

Les polymères associatifs amphotères sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon l'invention comprennent, ou sont préparés en copolymérisant :
1) au moins un monomère de formule (XIX) ou (XX) : 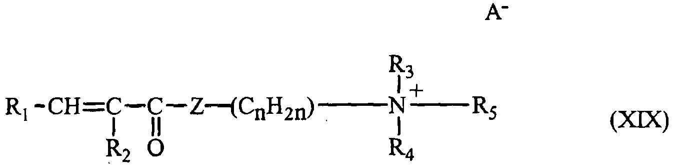 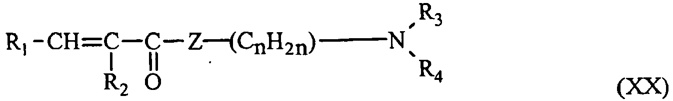
   dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A⁻ est un anion issu d'un acide organique ou minéral, tel qu'un anion méthosulfate ou un halogénure tel que chlorure ou bromure;
2) au moins un monomère de formule (XXI)

   R₆-CH=CR₇-COOH (XI)

   dans laquelle, R₆ et R₇, identiques ou différents, représentent un atome d'hydrogène ou un radical méthyle; et
3) au moins un monomère de formule (XXII) :

   R₆-CH=CR₇-COXR₈ (XXII)

   dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;

l'un au moins des monomères de formule (XIX), (XX) ou (XXII) comportant au moins une chaîne grasse.

Les monomères de formule (XIX) et (XX) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
ces monomères étant éventuellement quaternisés, par exemple par un halogénure d'alkyle en C₁₋C₄ ou un sulfate de dialkyle en C₁-C₄.

Plus particulièrement, le monomère de formule (XIX) est choisi parmi le chlorure d'acrylamidopropyl triméthyl ammonium et le chlorure de méthacrylamidopropyl triméthyl ammonium.

Les monomères de formule (XXI) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (XXI) est l'acide acrylique.

Les monomères de formule (XXII) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 % moles du monomère comportant une chaîne grasse (monomère de formule (XIX), (XX) ou (XXII)), et de préférence de 1,5 à 6% moles.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autres monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO98/44012.

Parmi les polymères associatifs amphotères selon l'invention, on préfère les terpolymères acide acrylique/chlorure de (méth)acrylamidopropyl triméthyl ammonium/ méthacrylate de stéaryle.

Les polymères associatifs de type non ionique utilisables selon l'invention sont choisis de préférence parmi :
- (1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212 ainsi que l'Acrysol RM 184®.

On peut également citer le produit ELFACOS T210® à chaîne alkyle en C₁₂₋₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéotate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Le ou les polymères épaississants additionnel(s) est ou sont présents dans la composition cosmétique selon l'invention en une quantité variant de 0,01 à 20 % en poids, de préférence en une quantité variant de 0,05 à 10 % en poids et encore plus préférentiellement en une quantité variant de 0,1 à 5 % en poids, par rapport au poids total de la composition.

La composition cosmétique selon l'invention peut comprendre en outre un ou plusieurs adjuvants cosmétiques choisis parmi les agents tensioactifs cationiques, anioniques, amphotères, non ioniques, les silicones autres que celles de l'invention, les agents conditionneurs de type esters, les agents anti-mousse, les agents hydratants, les agents émollients, les plastifiants, les filtres solaires hydrosolubles et liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents anti-corrosion, les corps gras tels que les huiles végétales, animales, minérales et synthétiques, les alcools gras, les agents réducteurs ou antioxydants, les agents oxydants.

L'homme du métier veillera à choisir les éventuels adjuvants et leur quantité de manière à ce qu'ils ne nuisent pas aux propriétés des compositions de la présente invention.

De préférence, le ou les adjuvants cosmétiques sont présents à une concentration allant de 0,001 à 50 % en poids par rapport au poids total de la composition.

Par milieu cosmétiquement acceptable, on entend un milieu compatible avec les matières kératiniques et en particulier les cheveux.

Le milieu cosmétiquement acceptable peut être un milieu alcoolique, aqueux ou hydroalcoolique. Ainsi le milieu peut notamment être constitué uniquement par de l'eau ou de l'alcool ou par un mélange d'eau et d'un ou de plusieurs solvants cosmétiquement acceptables tels que les alcools inférieurs en C₁-C₄, les polyols, les monoéthers de polyols et leurs mélanges. De préférence, l'alcool est l'éthanol.

Les compositions conformes à l'invention peuvent être conditionnées dans un pot, dans un tube, un flacon pompe ou encore dans un dispositif aérosol usuel en cosmétique.

Les agents propulseurs utilisés dans les systèmes aérosols selon l'invention peuvent être choisis parmi l'air, l'azote, le gaz carbonique, le diméthyléther, les alcanes en C₃ à C₅, le 1,1-difluoroéthane et leurs mélanges.

La présente invention concerne également un dispositif aérosol comprenant la composition décrite ci-dessus et un moyen de distribution de cette composition.

La présente invention concerne aussi un procédé de traitement cosmétique des cheveux, par exemple de coiffage, qui consiste à appliquer une quantité efficace d'une composition décrite ci-dessus, sur les cheveux secs ou humides, à rincer ou non après un éventuel temps de pose ou après un éventuel séchage.

La présente invention concerne également l'utilisation d'une composition cosmétique pour la mise en forme et/ou le maintien de la coiffure.

L'exemple suivant est donné à titre illustratif et non limitatif de la présente invention.

### EXEMPLE

On a préparé les compositions (A) à (D) conformes à l'invention à partir des composés suivants :

### Composition A

| | |
|---|---|
| - Terpolymère vinylpyrrolidone/ diméthylaminopropylméthacrylamide/ chlorure de lauryldiméthylméthacrylamidopropylammonium, proposé par la société ISP sous la dénomination Styleze W20 | 1 % |
| - Dispersion dans l'huile minérale d'homopolymère réticulé de chlorure de méthacryloyloxyéthyltriméthylammonium, vendu à 50 % en poids en homopolymère sous la dénomination « SALCARE SC 96 » par la Société ALLIED COLLOIDS | 1 % |
| - Amodiméthicone (DC 939) | 1 % |
| - Chlorure de cétyltriméthylammonium | 0,5 % |
| - Hydroxypropyl guar | 1 % |
| - Stéarate de glycérol | 1 % |
| - Polyvinylpyrrolidone | 2 % |
| - Conservateurs | qs |
| - Eau | 100% |

### Composition B

| | |
|---|---|
| - Terpolymère vinylpyrrolidone/ diméthylaminopropylméthacrylamide/ chlorure de lauryldiméthylméthacrylamidopropylammonium, proposé par la société ISP sous la dénomination Styleze W20 | 5 % |
| - Dispersion dans l'huile minérale d'homopolymère réticulé de chlorure de méthacryloyl-oxyéthyltriméthylammonium, vendu à 50 % en poids en homopolymère sous la dénomination « SALCARE SC 96 » par la Société ALLIED COLLOIDS | 2 % |
| - Amodiméthicone (DC 939) | 1 % |
| - Alcool cétéarylique | 4 % |
| - Chlorure de béhényltriméthylammonium | 1 % |
| - Glycérol | 3 % |
| - Conservateurs | qs |
| - Eau 100% | 100% |

### Composition C

| | |
|---|---|
| - Terpolymère vinylpyrrolidone/ diméthylaminopropylméthacrylamide/ chlorure de lauryldiméthylméthacrylamidopropylammonium, proposé par la société ISP sous la dénomination Styleze W20 | 4 % |
| - Dispersion dans l'huile minérale d'homopolymère réticulé de chlorure de méthacryloyl-oxyéthyltriméthylammonium, vendu à 50 % en poids en homopolymère sous la dénomination « SALCARE SC 96 » par la Société ALLIED COLLOIDS | 1 % |
| - Dimethiconol (DC1501 FLUID) | 1 % |
| - Phenyl triméthicone | 3 % |
| - Alcool cétéarylique | 4 % |
| - Chlorure de béhényltriméthylammonium | 1 % |
| - Laureth-4 | 0,5 % |
| - Huile minérale | 1 % |
| - Conservateurs | qs |
| -Eau | 100% |

### Composition D

| | |
|---|---|
| - Terpolymère vinylpyrrolidone/ diméthylaminopropylméthacrylamide/ chlorure de lauryldiméthylméthacrylamidopropylammonium, proposé par la société ISP sous la dénomination Styleze W20 | 1 % |
| - Dispersion dans l'huile minérale d'homopolymère réticulé de chlorure de méthacryloyloxyéthyltriméthylammonium, vendu à 50 % en poids en homopolymère sous la dénomination « SALCARE SC 96 » par la Société ALLIED COLLOIDS | 2 % |
| - Dimethiconol | 2 % |
| - Chlorure de cétyltriméthylammonium | 1 % |
| - Conservateurs | qs |
| - Eau | 100 % |

Les pourcentages de chacun des composés dans les compositions cosmétiques selon l'invention sont calculés en poids en matières actives par rapport au poids total de la composition.

Les compositions (A) à (D) sont appliquées sur des cheveux européens.

On constate que les compositions conformes à l'invention présentent de bonnes propriétés fixantes et confèrent également de très bonnes propriétés cosmétiques aux cheveux, en particulier en termes de douceur, de démêlage, d'aspect et de toucher.

## Revendications

1. Composition cosmétique pour le traitement des fibres kératiniques, en particulier des fibres kératiniques humaines telles que les cheveux, **caractérisée par le fait qu'**elle comprend, dans un milieu cosmétiquement acceptable,
- au moins un polymère poly(vinyllactame) cationique comprenant :
- a) au moins un monomère de type vinyl lactame ou alkylvinyllactame;
- b) au moins un monomère de structures (Ia) ou (Ib) suivantes : 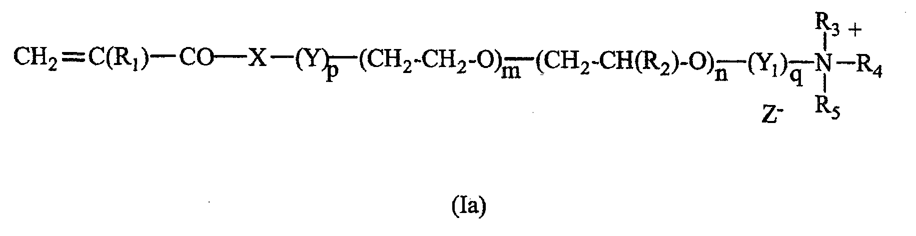 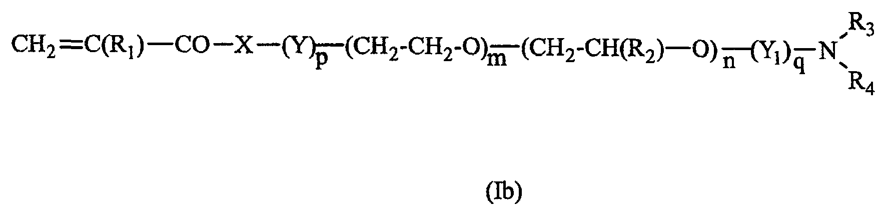 dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄,
R₃, R₄ et R₅ désignent, indépendamment l'un de l'autre, un atome d'hydrogène, un radical alkyle linéaire ou ramifié en C₁-C₃₀ ou un radical de formule (II) : 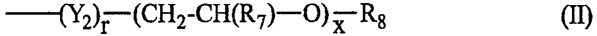
Y, Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0 ;
- au moins un polymère cationique réticulé choisi parmi les homo ou copolymères de chlorure de méthacryloyloxyalkyl(C₁-C₄)trialkyl(C₁-C₄) ammonium ; et
- au moins une silicone non volatile, en une quantité variant de 0,01 à 10% en poids, par rapport au poids total de la composition cosmétique.

2. Composition cosmétique selon la revendication 1, **caractérisée par le fait que** le monomère vinyl lactame ou alkylvinyllactame est un composé de structure (III) : 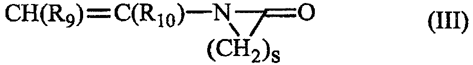 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

3. Composition cosmétique selon la revendication 2, **caractérisée par le fait que** le monomère de formule (III) est la vinylpyrrolidone.

4. Composition cosmétique selon l'une quelconque des revendications précédentes, **caractérisée par le fait que** dans les formules (Ia) ou (Ib), les radicaux R₃, R₄, R₅ désignent, indépendamment l'un de l'autre; un atome d'hydrogène ou un radical alkyle linéaire ou ramifié en C₁-C₃₀.

5. Composition cosmétique selon l'une quelconque des revendications précédentes, **caractérisée par le fait que** le monomère b) est un monomère de formule (la).

6. Composition cosmétique selon la revendication 5, **caractérisée par le fait que** dans la formule (Ia), m et n sont égaux à zéro.

7. Composition cosmétique selon l'une quelconque des revendications précédentes, **caractérisée par le fait que** le contre ion Z⁻ des monomères de formule (Ia) est choisi parmi les ions halogénures, les ions phosphates, l'ion méthosulfate, l'ion tosylate.

8. Composition cosmétique selon l'une quelconque des revendications 1 à 7, **caractérisée par le fait que** le ou les polymères poly(vinyllactame) cationiques contiennent un ou plusieurs monomères supplémentaires cationiques ou non ioniques.

9. Composition cosmétique selon la revendication 8, **caractérisée par le fait que** le poly(vinyllactame) cationique est un terpolymère comprenant :
a)-un monomère de formule (III),
b)-un monomère de formule (Ia) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (Ib) dans laquelle R₃ et R₄ désignent, indépendamment l'un de l'autre, un atome d'hydrogène ou un radical alkyle en C₁-C₅.

10. Composition cosmétique selon la revendication 9, **caractérisée par le fait que** le terpolymère comprend en poids, 40 à 95% de monomère (a), 0,25 à 50% de monomère (b) et 0,1 à 55% de monomère (c).

11. Composition selon l'une quelconque des revendications précédentes, **caractérisée par le fait que** les poly(vinyllactame) cationiques sont choisis parmi les terpolymères vinylpyrrolidone / diméthylaminopropylméthacrylamide / tosylate de dodécyldiméthylméthacrylamidopropylammonium, les terpolymères vinylpyrrolidone / diméthylaminopropylméthacrylamide / tosylate de cocoyldiméthylméthacrylamidopropylammonium, les terpolymères vinylpyrrolidone / diméthylaminopropylméthacrylamide / tosylate ou chlorure de lauryldiméthylméthacrylamidopropylammonium.

12. Composition selon l'une quelconque des revendications précédentes, **caractérisée par le fait que** ledit polymère poly(vinyllactame) cationique est un terpolymère vinylpyrrolidone / diméthylaminopropylméthacrylamide / chlorure de lauryldiméthyl-méthacrylamidopropylammonium.

13. Composition selon l'une quelconque des revendications précédentes, **caractérisée par le fait que** la masse moléculaire en poids des poly(vinyllactame) cationiques est comprise entre 500 et 20 000 000, de préférence comprise entre 200 000 et 2 000 000 et plus préférentiellement comprise entre 400 000 et 800 000.

14. Composition cosmétique selon l'une quelconque des revendications précédentes, **caractérisée par le fait que** le ou les poly(vinyllactame) cationiques sont utilisés en une quantité variant de 0,05 à 30 % en poids, de préférence en une quantité variant de 0,1 à 15 % en poids et encore plus préférentiellement en une quantité variant de 0,2 à 10 % en poids, par rapport au poids total de la composition.

15. Composition cosmétique selon l'une quelconque des revendications précédentes, **caractérisée par le fait que** ledit polymère cationique réticulé est un polymère réticulé de chlorure de méthacryloyloxyéthyl triméthylammonium de type homopolymère ou copolymère avec l'acrylamide.

16. Composition cosmétique selon la revendication 15, **caractérisée par le fait que** le copolymère d'acrylamide / chlorure de méthacryloyloxyéthyl triméthylammonium est obtenu par copolymérisation de l'acrylamide et du diméthylaminoéthyl-méthacrylate quaternisé par le chlorure de méthyle et que l'homopolymère est obtenu par homopolymérisation du diméthylaminoéthylméthacrylate quaternisé par le chlorure de méthyle, la polymérisation étant suivie d'une réticulation par un composé à insaturation oléfinique, en particulier le méthylène-bis-acrylamide.

17. Composition cosmétique selon l'une quelconque des revendications 1 à 16, **caractérisée par** le fait ledit polymère cationique réticulé est un copolymère réticulé acrylamide / chlorure de méthacryloyloxyéthyl triméthylammonium (20/80 en poids) sous forme de dispersion contenant 50 % en poids dudit copolymère dans de l'huile minérale.

18. Composition cosmétique selon l'une quelconque des revendications 1 à 16, **caractérisée par le fait que** ledit polymère cationique réticulé est un homopolymère réticulé de chlorure de méthacryloyloxyéthyl triméthylammonium sous forme de dispersion contenant 50 % en poids dudit homopolymère dans de l'huile minérale.

19. Composition cosmétique selon l'une quelconque des revendications précédentes, **caractérisée par le fait que** le ou les polymère(s) cationique(s) réticulé(s) choisi(s) parmi les homo ou copolymères de chlorure de méthacryloyloxyalkyl(C₁-C₄)trialkyl(C₁-C₄) ammonium est ou sont présents en une quantité variant de 0,01 à 30 % en poids, de préférence en une quantité variant de 0,05 à 20 % en poids et encore plus préférentiellement en une quantité variant de 0,1 à 10 % en poids, par rapport au poids total de la composition cosmétique.

20. Composition cosmétique selon l'une quelconque des revendications précédentes, **caractérisée par le fait que** la silicone non volatile est choisie parmi les polyorganosiloxanes insolubles dans la composition.

21. Composition cosmétique selon la revendication 20, **caractérisée par le fait que** les polyorganosiloxanes sont des polyorganosiloxanes non volatils choisis parmi les polyalkylsiloxanes, des polyarylsiloxanes, des polyalkylarylsiloxanes, des gommes et résines de silicones, des polyorganosiloxanes modifiés par des groupements organofonctionnels ainsi que leurs mélanges.

22. Composition cosmétique selon la revendication 21, **caractérisée par le fait que** :
(a) les polyalkylsiloxanes sont choisi parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les polyalkylarylsiloxanes sont choisi parmi :
- les polydiméthylméthylphénylsiloxanes, les polydiméthyl diphénylsiloxanes linéaires et/ou ramifiés de viscosité de 1.10⁻⁵ à 5.10⁻² m²/s à 25°C.
(c) les gommes de silicone sont choisies parmi les polydiorganosiloxanes ayant des masses moléculaires moyennes en nombre comprises entre 200 000 et 1000000 utilisés seuls ou sous forme de mélange dans un solvant ;
(d) les résines sont choisies parmi les résines constituées d'unités : R₃SiO_{1/2}, R₂SiO_{2/2}, RSiO_{3/2}, SiO_{4/2}
dans lesquelles R représente un groupement hydrocarboné possédant de 1 à 16 atomes de carbone ou un groupement phényle ;
(e) les silicones organomodifiées sont choisies parmi les silicones comportant dans leur structure un ou plusieurs groupements organofonctionnels fixés par l'intermédiaire d'un radical hydrocarboné.

23. Composition cosmétique selon la revendication 22, **caractérisée par le fait que** les gommes de silicone utilisées seules ou sous forme de mélange sont choisies parmi les structures suivantes :
- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane et les mélanges suivants :
- des mélanges formés à partir d'un polydiméthylsiloxane hydroxylé en bout de chaîne et d'un polydiméthylsiloxane cyclique ;
- des mélanges formés à partir d'une gomme polydiméthylsiloxane et d'une silicone cyclique ;
- des mélanges de polydiméthylsiloxanes de viscosités différentes.

24. Composition cosmétique selon la revendication 22, **caractérisée par le fait que** les silicones organomodifées sont choisies parmi les polyorganosiloxanes comportant :
- des groupements polyéthylèneoxy et/ou polypropylèneoxy
- des groupements aminés substitués ou non ;
- des groupements thiols ;
- des groupements alcoxylés ;
- des groupements hydroxyalkyle ;
- des groupements acyloxyalkyle ;
- des groupements alkylcarboxyliques ;
- des groupements 2-hydroxyalkylsulfonates ;
- des groupements 2-hydroxyalkylthiosulfonates ;
- des groupements hydroxyacylamino.

25. Composition cosmétique selon l'une quelconque des revendications 21 à 24, **caractérisée par le fait que** les polyorganosiloxanes sont choisis parmi les polyalkylsiloxanes à groupements terminaux triméthylsilyle, les polyalkylsiloxanes à groupements terminaux diméthylsilanol, les polyalkylarylsiloxanes, les mélanges de deux polydiméthylsiloxanes constituées d'une gomme et d'une huile de viscosités différentes, les mélanges d'organosiloxanes et de silicones cycliques, les résines d'organopolysiloxanes.

26. Composition cosmétique selon l'une quelconque des revendications précédentes, **caractérisée par le fait que** la silicone non volatile est présente en une quantité variant de 0,05 à 10 % en poids, et encore plus préférentiellement en une quantité variant de 0,1 à 10 % en poids, par rapport au poids total de la composition cosmétique.

27. Composition cosmétique selon l'une quelconque des revendications précédentes, **caractérisée par le fait que** le rapport pondéral poly(vinyllactame) cationique / polymère cationique réticulé est compris entre 0,1 et 10.

28. Composition cosmétique selon l'une quelconque des revendications précédentes, **caractérisée par le fait que** le rapport pondéral poly(vinyllactame) cationique / silicone non volatile est compris entre 0,1 et 10.

29. Composition cosmétique selon l'une quelconque des revendications précédentes, **caractérisée par le fait que** le rapport pondéral polymère cationique réticulé / silicone non volatile est compris entre 0,2 et 5.

30. Composition cosmétique selon l'une quelconque des revendications précédentes, **caractérisée par le fait qu'**elle comprend en outre au moins un polymère fixant additionnel.

31. Composition cosmétique selon l'une quelconque des revendications précédentes, **caractérisée par le fait qu'**elle comprend en outre au moins un polymère épaississant.

32. Composition cosmétique selon l'une quelconque des revendications précédentes, **caractérisée par le fait qu'**elle comprend en outre au moins un adjuvant cosmétique choisi parmi les agents tensioactifs cationiques, anioniques, amphotères, non ioniques, les agents conditionneurs de type esters, les agents anti-mousse, les agents hydratants, les agents émollients, les plastifiants, les filtres solaires hydrosolubles et liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents anti-corrosion, les corps gras tels que les huiles végétales, animales, minérales et synthétiques, les alcools gras, les agents réducteurs ou antioxydants, les agents oxydants.

33. Composition cosmétique selon la revendication 32, **caractérisée par le fait que** le ou les adjuvants cosmétiques sont présents à une concentration allant de 0,001 à 50 % en poids par rapport au poids total de la composition.

34. Composition cosmétique selon l'une quelconque des revendications précédentes, **caractérisée par le fait que** le milieu cosmétiquement acceptable est un milieu aqueux, alcoolique ou hydroalcoolique.

35. Composition cosmétique selon la revendication 34, **caractérisée par le fait que** le milieu hydroalcoolique comprend les alcools inférieurs en C₁-C₄, les polyols, les monoéthers de polyols et leurs mélanges.

36. Composition cosmétique selon la revendication 35, **caractérisée par le fait que** l'alcool est de l'éthanol.

37. Composition cosmétique selon l'une quelconque des revendications précédentes, **caractérisée par le fait qu'**elle est conditionnée dans un vaporisateur, un flacon pompe ou un dispositif aérosol.

38. Composition cosmétique selon la revendication 37, **caractérisée par le fait qu'**elle est conditionnée dans un dispositif aérosol.

39. Composition cosmétique selon la revendication 38, **caractérisée par le fait qu'**elle comprend un agent propulseur choisi parmi l'air, l'azote, le gaz carbonique, le diméthyléther, les alcanes en C₃ à C₅, le 1,1-difluoroéthane et leurs mélanges.

40. Dispositif aérosol formé par un récipient comprenant une composition selon l'une quelconque des revendications précédentes ainsi qu'un moyen de distribution de la composition.

41. Procédé de traitement cosmétique **caractérisée par le fait qu'**il comprend l'application d'une composition cosmétique selon l'une quelconque des revendications 1 à 39, en particulier sur les cheveux.

42. Procédé de traitement cosmétique selon la revendication 41, **caractérisée par le fait que** l'application de ladite composition n'est pas suivie par un rinçage.

43. Utilisation d'une composition cosmétique selon l'une quelconque des revendications 1 à 39 pour la mise en forme et/ou le maintien de la coiffure.

## Patentansprüche

1. Kosmetische Zusammensetzung für die Behandlung von Keratinfasern, insbesondere von menschlichen Keratinfasern wie dem Haar, **dadurch gekennzeichnet, dass** sie in einem kosmetisch unbedenklichen Medium Folgendes umfasst:
- mindestens ein kationisches Poly(vinyllactam)-polymer, das Folgendes umfasst:
- a) mindestens ein Monomer des Vinyllactam- oder Alkylvinyllactamtyps;
- b) mindestens ein Monomer mit den folgenden Strukturen (Ia) oder (Ib): 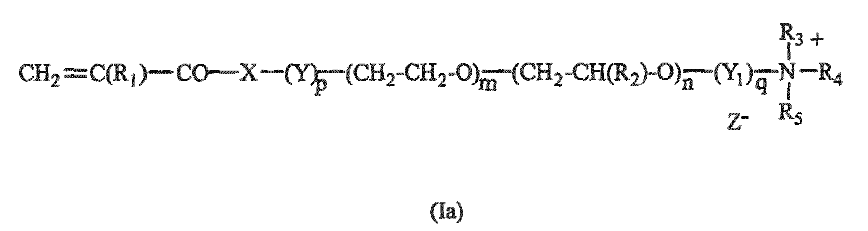 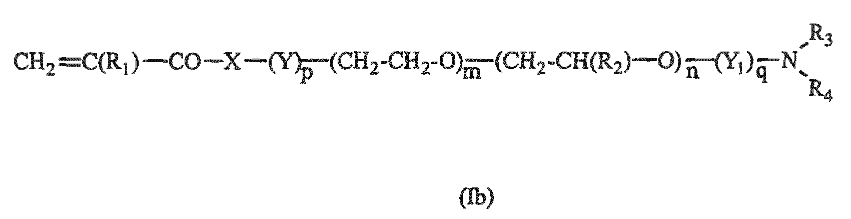 in denen:
X ein Sauerstoffatom oder einen NR₆-Rest bedeutet,
R₁ und R₆ unabhängig voneinander ein Wasserstoffatom oder einen geradkettigen oder
verzweigten C₁-C₅-Alkylrest bedeuten,
R₂ einen geradkettigen oder verzweigten C₁-C₄-Alkylrest bedeutet,
R₃, R₄ und R₅ unabhängig voneinander ein Wasserstoffatom, einen geradkettigen oder
verzweigten C₁-C₃₀-Alkylrest oder einen Rest der Formel (II) : 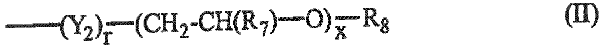 bedeuten,
Y, Y₁ und Y₂ unabhängig voneinander einen geradkettigen oder verzweigten C₂-C₁₆-Alkylenrest bedeuten,
R₇ ein Wasserstoffatom, einen geradkettigen oder verzweigten C₁-C₄-Alkylrest oder einen geradkettigen oder verzweigten C₁-C₄-Hydroxyalkylrest bedeutet,
R₈ ein Wasserstoffatom oder einen geradkettigen oder verzweigten C₁-C₃₀-Alkylrest bedeutet, p, q und r unabhängig voneinander den Wert 0 oder den Wert 1 bedeuten,
m und n unabhängig voneinander eine ganze Zahl von 0 bis 100 bedeuten,
x eine ganze Zahl von 1 bis 100 bedeutet,
Z ein Anion einer organischen oder anorganischen Säure bedeutet,
mit der Maßgabe, dass:
- mindestens einer der Substituenten R₃, R₄, R₅ oder R₈ einen geradkettigen oder verzweigten C₉-C₃₀-Alkylrest bedeutet,
- dann, wenn m oder n von null verschieden sind, q 1 ist,
- dann, wenn m oder n gleich null sind, p oder q gleich 0 ist;
- mindestens ein vernetztes kationisches Polymer, das aus den Homo- oder Copolymeren von Methacryloyloxy (C₁-C₄)alkyltri(C₁-C₄)alkylammonium-chlorid ausgewählt ist; und
- mindestens ein nichtflüchtiges Silikon in einer Menge von 0,01 bis 10 Gew.-%, bezogen auf das Gesamtgewicht der kosmetischen Zusammensetzung.

2. Kosmetische Zusammensetzung nach Anspruch 1, **dadurch gekennzeichnet, dass** es sich bei dem Vinyllactam- oder Alkylvinyllactammonomer um eine Verbindung der folgenden Struktur (III) handelt: 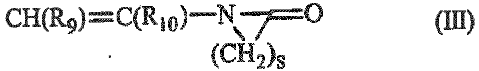 in der
s eine ganze Zahl von 3 bis 6 bedeutet,
R₉ ein Wasserstoffatom oder einen C₁-C₅-Alkylrest bedeutet,
R₁₀ ein Wasserstoffatom oder einen C₁-C₅-Alkylrest bedeutet,
mit der Maßgabe, dass mindestens einer der Reste Rg und R₁₀ ein Wasserstoffatom bedeutet.

3. Kosmetische Zusammensetzung nach Anspruch 2, **dadurch gekennzeichnet, dass** es sich bei dem Monomer der Formel (III) um Vinylpyrrolidon handelt.

4. Kosmetische Zusammensetzung nach einem der vorhergehenden Ansprüche, **dadurch gekennzeichnet, dass** die Reste R₃, R₄, R₅ in den Formeln (Ia) bzw. (Ib) unabhängig voneinander ein Wasserstoffatom oder einen geradkettigen oder verzweigten C₁-C₃₀-Alkylrest bedeuten.

5. Kosmetische Zusammensetzung nach einem der vorhergehenden Ansprüche, **dadurch gekennzeichnet, dass** es sich bei dem Monomer b) um ein Monomer der Formel (Ia) handelt.

6. Kosmetische Zusammensetzung nach Anspruch 5, **dadurch gekennzeichnet, dass** m und n in Formel (Ia) gleich null sind.

7. Kosmetische Zusammensetzung nach einem der vorhergehenden Ansprüche, **dadurch gekennzeichnet, dass** das Gegenion Z⁻ der Monomere der Formel (Ia) aus Halogenidionen, Phosphationen, Methosulfation, Tosylation ausgewählt ist.

8. Kosmetische Zusammensetzung nach einem der Ansprüche 1 bis 7, **dadurch gekennzeichnet, dass** das kationische Poly(vinyllactam)polymer bzw. die kationischen Poly(vinyllactam)polymere ein oder mehrere zusätzliche kationische oder nichtionische Monomere enthalten.

9. Kosmetische Zusammensetzung nach Anspruch 8, **dadurch gekennzeichnet, dass** es sich bei dem kationischen Poly(vinyllactam) um ein Terpolymer handelt, das Folgendes umfasst:
a)- ein Monomer der Formel (III),
b)- ein Monomer der Formel (Ia), worin p=1, q=0, R₃ und R₄ unabhängig voneinander ein Wasserstoffatom oder einen C₁-C₅-Alkylrest bedeuten und R₅ einen C₉-C₂₄-Alkylrest bedeutet, und
c)- ein Monomer der Formel (Ib), worin R₃ und R₄ unabhängig voneinander ein Wasserstoffatom oder einen C₁-C₅-Alkylrest bedeuten.

10. Kosmetische Zusammensetzung nach Anspruch 9, **dadurch gekennzeichnet, dass** das Terpolymer 40 bis 95 Gew.-% Monomer (a), 0,25 bis 50 Gew.-% Monomer (b) und 0,1 bis 55 Gew.-% Monomer (c) umfasst.

11. Zusammensetzung nach einem der vorhergehenden Ansprüche, **dadurch gekennzeichnet, dass** die kationischen Poly(vinyllactame) aus Vinylpyrrolidon/Dimethylaminopropylmethacrylamid/Dodecyldimethylmethacrylamidopropylammoniumtosylat-Terpolymeren, Vinylpyrrolidon/Dimethylaminopropylmethacrylamid/Cocoyldimethylmethacrylamidopropylammoniumtosylat-Terpolymeren, Vinylpyrrolidon/Dimethylaminopropylmethacrylamid/Lauryldimethylmethacrylamidopropylammoniumtosylat- oder -chlorid-Terpolymeren ausgewählt sind.

12. Zusammensetzung nach einem der vorhergehenden Ansprüche, **dadurch gekennzeichnet, dass** es sich bei dem kationischen Poly(vinyllactam)polymer um ein Vinylpyrrolidon/Dimethylaminopropylmethacrylamid/Lauryldimethylmethacrylamidopropylammoniumchlorid-Terpolymer handelt.

13. Zusammensetzung nach einem der vorhergehenden Ansprüche, **dadurch gekennzeichnet, dass** das gewichtsmittlere Molekulargewicht der kationischen Poly(vinyllactame) zwischen 500 und 20.000.000, vorzugsweise zwischen 200.000 und 2.000.000 und weiter bevorzugt zwischen 400.000 und 800.000 liegt.

14. Kosmetische Zusammensetzung nach einem der vorhergehenden Ansprüche, **dadurch gekennzeichnet, dass** das kationische Poly(vinyllactam) bzw. die kationischen Poly(vinyllactame) in einer Menge von 0,05 bis 30 Gew.-%, vorzugsweise in einer Menge von 0,1 bis 15 Gew.-% und noch weiter bevorzugt in einer Menge von 0,2 bis 10 Gew.-%, bezogen auf das Gesamtgewicht der Zusammensetzung, verwendet wird bzw. werden.

15. Kosmetische Zusammensetzung nach einem der vorhergehenden Ansprüche, **dadurch gekennzeichnet, dass** es sich bei dem vernetzten kationischen Polymer um ein vernetztes Methacryloyloxyethyltrimethylammoniumchlorid-Polymer vom Homopolymer- oder Acrylamid-Copolymer-Typ handelt.

16. Kosmetische Zusammensetzung nach Anspruch 15, **dadurch gekennzeichnet, dass** das Acrylamid/Methacryloyloxyethyltrimethylammoniumchlorid-Copolymer durch Copolymerisation von Acrylamid und mit Methylchlorid quaternisiertem Dimethylaminoethylmethacrylat erhalten wird und das Homopolymer durch Homopolymerisation von mit Methylchlorid quaternisiertem Dimethylaminoethylmethacrylat erhalten wird, wobei auf die Polymerisation eine Vernetzung mit einer olefinisch ungesättigten Verbindung, insbesondere Methylenbisacrylamid, folgt.

17. Kosmetische Zusammensetzung nach einem der Ansprüche 1 bis 16, **dadurch gekennzeichnet, dass** es sich bei dem vernetzten kationischen Polymer um ein vernetztes Acrylamid/Methacryloyloxy(ethyl-trimethylammoniumchlorid-Copolymer (Gewichtsverhältnis 20/80) in Form einer Dispersion, die 50 Gew.-% des Copolymers in Mineralöl enthält, handelt.

18. Kosmetische Zusammensetzung nach einem der Ansprüche 1 bis 16, **dadurch gekennzeichnet, dass** es sich bei dem vernetzten kationischen Polymer um ein vernetztes Methacryloyloxyethyltrimethylammoniumchlorid-Homopolymer in Form einer Dispersion, die 50 Gew.-% des Homopolymers in Mineralöl enthält, handelt.

19. Kosmetische Zusammensetzung nach einem der vorhergehenden Ansprüche, **dadurch gekennzeichnet, dass** das vernetzte kationische Polymer bzw. die vernetzten kationischen Polymere, das bzw. die aus Methacryloyloxy(C₁-C₄)alkyltri(C₁-C₄)alkylammonium-Homo- oder -Copolymeren ausgewählt ist bzw. sind, in einer Menge von 0,01 bis 30 Gew.-%, vorzugsweise in einer Menge von 0,05 bis 20 Gew.-% und noch weiter bevorzugt in einer Menge von 0,1 bis 10 Gew.-%, bezogen auf das Gesamtgewicht der kosmetischen Zusammensetzung, vorliegt bzw. vorliegen.

20. Kosmetische Zusammensetzung nach einem der vorhergehenden Ansprüche, **dadurch gekennzeichnet, dass** das nichtflüchtige Silikon aus in der Zusammensetzung unlöslichen Polyorganosiloxanen ausgewählt ist.

21. Kosmetische Zusammensetzung nach Anspruch 20, **dadurch gekennzeichnet, dass** es sich bei den Polyorganosiloxanen um nichtflüchtige Polyorganosiloxane, die aus Polyalkylsiloxanen, Polyarylsiloxanen, Polyalkylarylsiloxanen, Silikongummis und -harzen, mit organofunktionellen Gruppen modifizierten Polyorganosiloxanen sowie Mischungen davon ausgewählt sind, handelt.

22. Kosmetische Zusammensetzung nach Anspruch 21, **dadurch gekennzeichnet, dass**:
(a) die Polyalkylsiloxane ausgewählt sind aus:
- Polydimethylsiloxanen mit Trimethylsilyl-Endgruppen;
- Polydimethylsiloxanen mit Dimethylsilanol-Endgruppen;
- Poly(C₁-C₂₀)alkylsiloxanen;
(b) die Polyalkylarylsiloxane ausgewählt sind aus: - Polydimethylmethylphenylsiloxanen und linearen und/oder verzweigten Polydimethyldiphenylsiloxanen mit einer Viskosität von 1.10⁻⁵ bis 5.10⁻² m²/s bei 25°C;
(c) die Silikongummis ausgewählt sind aus Polydiorganosiloxanen mit zahlenmittleren Molekulargewichten von 200.000 bis 1.000.000, die alleine oder in Form einer Mischung in einem Lösungsmittel verwendet werden;
(d) die Harze ausgewählt sind aus Harzen, die aus R₃SiO_{1/2}-, R₂SiO_{2/2}-, RSiO_{3/2}-, SiO_{4/2}-Einheiten bestehen,
wobei R für eine Kohlenwasserstoffgruppe mit 1 bis 16 Kohlenstoffatomen oder eine Phenylgruppe steht;
(e) die organomodifizierten Silikone ausgewählt sind aus Silikonen, die in ihrer Struktur eine oder mehrere organofunktionelle Gruppen, die über einen Kohlenwasserstoffrest gebunden sind, enthalten.

23. Kosmetische Zusammensetzung nach Anspruch 22, **dadurch gekennzeichnet, dass** die allein oder in Form einer Mischung verwendeten Silikongummis aus den folgenden Strukturen:
- Polydimethylsiloxan,
- Polydimethylsiloxan/methylvinylsiloxan-Gummis,
- Polydimethylsiloxan/diphenylmethylsiloxan,
- Polydimethylsiloxan/phenylmethylsiloxan,
- Polydimethylsiloxan/diphenylsiloxan/methylvinylsiloxan und den folgenden Mischungen:
- Mischungen aus einem am Kettenende hydroxylierten Polydimethylsiloxan und einem cyclischen Polydimethylsiloxan;
- Mischungen aus einem Polydimethylsiloxangummi und einem cyclischen Silikon;
- Mischungen aus Polydimethylsiloxanen mit verschiedenen Viskositäten
ausgewählt sind.

24. Kosmetische Zusammensetzung nach Anspruch 22, **dadurch gekennzeichnet, dass** die organomodifizierten Silikone aus den Polyorganosiloxanen mit:
- Polyethylenoxy- und/oder Polypropylenoxygruppen;
- substituierten oder unsubstituierten Aminogruppen;
- Thiolgruppen;
- Alkoxygruppen;
- Hydroxyalkylgruppen;
- Acyloxyalkylgruppen;
- Alkylcarboxylgruppen
- 2-Hydroxyalkylsulfonatgruppen;
- 2-Hydroxyalkylthiosulfonatgruppen;
- Hydroxyacylaminogruppen
ausgewählt sind.

25. Kosmetische Zusammensetzung nach einem der Ansprüche 1 bis 24, **dadurch gekennzeichnet, dass** die Polyorganosiloxane aus Polyalkylsiloxanen mit Trimethylsilyl-Endgruppen, Polyalkylsiloxanen mit Dimethylsilanol-Endgruppen, Polyalkylarylsiloxanen, Mischungen von zwei Polydimethylsiloxanen, die aus einem Gummi und einem Öl mit verschiedenen Viskositäten bestehen, Mischungen von Organosiloxanen und cyclischen Silikonen, Organopolysiloxanharzen ausgewählt sind.

26. Kosmetische Zusammensetzung nach einem der vorhergehenden Ansprüche, **dadurch gekennzeichnet, dass** das nichtflüchtige Silikon in einer Menge von 0,05 bis 10 Gew.-% und noch weiter bevorzugt in einer Menge von 0,1 bis 10 Gew.-%, bezogen auf das Gesamtgewicht der kosmetischen Zusammensetzung, vorliegt.

27. Kosmetische Zusammensetzung nach einem der vorhergehenden Ansprüche, **dadurch gekennzeichnet, dass** das Gewichtsverhältnis von kationischem Poly(vinyllactam) zu vernetztem kationischen Polymer zwischen 0,1 und 10 liegt.

28. Kosmetische Zusammensetzung nach einem der vorhergehenden Ansprüche, **dadurch gekennzeichnet, dass** das Gewichtsverhältnis von kationischem Poly(vinyllactam) zu nichtflüchtigem Silikon zwischen 0,1 und 10 liegt.

29. Kosmetische Zusammensetzung nach einem der vorhergehenden Ansprüche, **dadurch gekennzeichnet, dass** das Gewichtsverhältnis von vernetztem kationischen Polymer zu nichtflüchtigem Silikon zwischen 0,2 und 5 liegt.

30. Kosmetische Zusammensetzung nach einem der vorhergehenden Ansprüche, **dadurch gekennzeichnet, dass** sie weiterhin mindestens ein zusätzliches fixierendes Polymer umfasst.

31. Kosmetische Zusammensetzung nach einem der vorhergehenden Ansprüche, **dadurch gekennzeichnet, dass** sie weiterhin mindestens ein verdickendes Polymer umfasst.

32. Kosmetische Zusammensetzung nach einem der vorhergehenden Ansprüche, **dadurch gekennzeichnet, dass** sie weiterhin mindestens einen kosmetischen Hilfsstoff, der aus kationischen Tensiden, anionischen Tensiden, amphoteren Tensiden, nichtionischen Tensiden, Konditionierungsmitteln vom Estertyp, Antischaummitteln, feuchtigkeitsspendenden Mitteln, zartmachenden Mitteln, Weichmachern, silikonhaltigen oder nicht silikonhaltigen wasserlöslichen und fettlöslichen Sonnenschutzfiltern, permanenten oder temporären Farbstoffen, Duftstoffen, Peptisierungsmitteln, Konservierungsmitteln, Ceramiden und Pseudoceramiden, Vitaminen und Provitaminen, darunter Panthenol, Proteinen, Sequestriermitteln, Hilfslösungsmitteln, Alkanisierungsmitteln, Korrosionsschutzmitteln, Fettsubstanzen wie pflanzlichen, tierischen, mineralischen und synthetischen Ölen, Fettalkoholen, Reduktionsmitteln oder Antioxidantien, Oxidationsmitteln ausgewählt ist, umfasst.

33. Kosmetische Zusammensetzung nach Anspruch 32, **dadurch gekennzeichnet, dass** der kosmetische Hilfsstoff bzw. die kosmetischen Hilfsstoffe in einer Konzentration im Bereich von 0,001 bis 50 Gew.-%, bezogen auf das Gesamtgewicht der Zusammensetzung, vorliegt bzw. vorliegen.

34. Kosmetische Zusammensetzung nach einem der vorhergehenden Ansprüche, **dadurch gekennzeichnet, dass** es sich bei dem kosmetisch unbedenklichen Medium um ein wässriges, alkoholisches oder wässrig-alkoholisches Medium handelt.

35. Kosmetische Zusammensetzung nach Anspruch 34, **dadurch gekennzeichnet, dass** das wässrigalkoholische Medium niedere C₁-C₄-Alkohle, Polyole, Polyolmonoether und Mischungen davon umfasst.

36. Kosmetische Zusammensetzung nach Anspruch 35, **dadurch gekennzeichnet, dass** es sich bei dem Alkohol um Ethanol handelt.

37. Kosmetische Zusammensetzung nach einem der vorhergehenden Ansprüche, **dadurch gekennzeichnet, dass** sie in einer Sprühflasche, einer Pumpflasche oder einer Aerosolvorrichtung verpackt ist.

38. Kosmetische Zusammensetzung nach Anspruch 37, **dadurch gekennzeichnet, dass** sie in einer Aerosolvorrichtung verpackt ist.

39. Kosmetische Zusammensetzung nach Anspruch 38, **dadurch gekennzeichnet, dass** sie ein Treibmittel, das aus Luft, Stickstoff, Kohlendioxid, Dimethylether, C₃- bis C₅-Alkanen, 1,1-Difluorethan und Mischungen davon ausgewählt ist, umfasst.

40. Aerosolvorrichtung, gebildet aus einem Behältnis, das eine Zusammensetzung nach einem der vorhergehenden Ansprüche umfasst, sowie einem Mittel zum Verteilen der Zusammensetzung.

41. Verfahren zur kosmetischen Behandlung, **dadurch gekennzeichnet, dass** man eine Zusammensetzung nach einem der Ansprüche 1 bis 39 aufbringt, insbesondere auf das Haar.

42. Verfahren zur kosmetischen Behandlung nach Anspruch 41, **dadurch gekennzeichnet, dass** nach dem Aufbringen der Zusammensetzung nicht gespült wird.

43. Verwendung einer Zusammensetzung nach einem der Ansprüche 1 bis 39 zum Gestalten und/oder Festigen der Frisur.

## Claims

1. Cosmetic composition for treating keratin fibres, in particular human keratin fibres such as the hair, **characterized in that** it comprises, in a cosmetically acceptable medium,
- at least one cationic poly(vinyllactam) polymer comprising:
a) at least one monomer of vinyllactam or alkylvinyllactam type;
b) at least one monomer of the following structures (Ia) or (Ib): 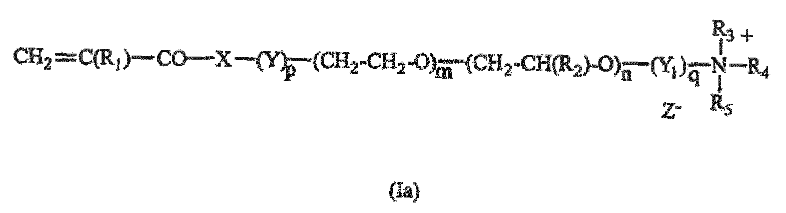 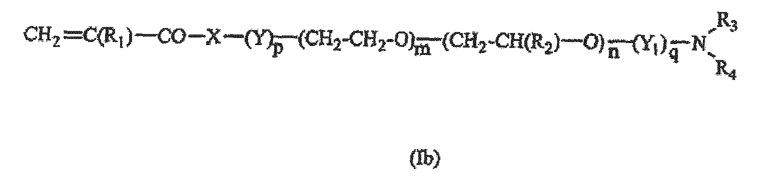 in which:
X denotes an oxygen atom or a radical NR₆,
R₁ and R₆ denote, independently of one another, a hydrogen atom or a linear or branched C₁-C₅ alkyl radical,
R₂ denotes a linear or branched C₁-C₄ alkyl radical,
R₃, R₄ and R₅ denote, independently of one another, a hydrogen atom, a linear or branched C₁-C₃₀ alkyl radical, or a radical of formula (II): 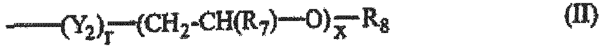
Y, Y₁ and Y₂ denote, independently of one another, a linear or branched C₂-C₁₆ alkylene radical,
R₇ denotes a hydrogen atom or a linear or branched C₁-C₄ alkyl radical or a linear or branched C₁-C₄ hydroxyalkyl radical,
R₈ denotes a hydrogen atom or a linear or branched C₁-C₃₀ alkyl radical,
p, q and r denote, independently of one another, either 0 or 1,
m and n denote, independently of one another, an integer ranging from 0 to 100,
x denotes an integer ranging from 1 to 100,
Z denotes an anion of an organic or inorganic acid,
with the proviso that:
- at least one of the substituents R₃, R₄, R₅ or R₈ denotes a linear or branched C₉-C₃₀ alkyl radical,
- if m or n is other than 0, then q is equal to 1,
- if m or n is equal to zero, then p or q is equal to 0;
- at least one crosslinked cationic polymer chosen from the homopolymers or copolymers of methacryloyloxy(C₁-C₄) alkyltri(C₁-C₄) alkylammonium chloride; and
- at least one nonvolatile silicone in an amount ranging from 0.01 to 10% by weight relative to the total weight of the cosmetic composition.

2. Cosmetic composition according to Claim 1, **characterized in that** the vinyllactam or alkylvinyllactam monomer is a compound of structure (III): 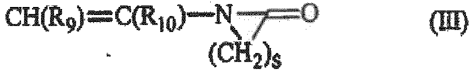 in which:
s denotes an integer ranging from 3 to 6,
R₉ denotes a hydrogen atom or a C₁-C₅ alkyl radical,
R₁₀ denotes a hydrogen atom or a C₁-C₅ alkyl radical,
with the proviso that at least one of the radicals R₉ and R₁₀ denotes a hydrogen atom.

3. Cosmetic composition according to Claim 2, **characterized in that** the monomer of formula (III) is vinylpyrrolidone.

4. Cosmetic composition according to any one of the preceding claims, **characterized in that** in formulae (Ia) or (Ib) the radicals R₃, R₄ and R₅ denote, independently of one another, a hydrogen atom or a linear or branched C₁-C₃₀ alkyl radical.

5. Cosmetic composition according to any one of the preceding claims, **characterized in that** the monomer b) is a monomer of formula (Ia).

6. Cosmetic composition according to Claim 5, **characterized in that** in formula (Ia) m and n are equal to 0.

7. Cosmetic composition according to any one of the preceding claims, **characterized in that** the counterion Z⁻ of the monomers of formula (Ia) is chosen from halide ions, phosphate ions, the methosulfate ion and the tosylate ion.

8. Cosmetic composition according to any one of Claims 1 to 7, **characterized in that** the cationic poly(vinyllactam) polymer(s) contain(s) one or more additional cationic or nonionic monomers.

9. Cosmetic composition according to Claim 8, **characterized in that** the cationic poly(vinyllactam) is a terpolymer comprising:
a) a monomer of formula (III),
b) a monomer of formula (Ia), in which p=1, q=0, R₃ and R4 denote, independently of one another, a hydrogen atom or a C₁-C₅ alkyl radical, and R₅ denotes a C₉-C₂₄ alkyl radical, and
c) a monomer of formula (Ib), in which R₃ and R₄ denote, independently of one another, a hydrogen atom or a C₁-C₅ alkyl radical.

10. Cosmetic composition according to Claim 9, **characterized in that** the terpolymer comprises, by weight, 40 to 95% of monomer (a), 0.25 to 50% of monomer (b) and 0.1 to 55% of monomer (c).

11. Composition according to any one of the preceding claims, **characterized in that** the cationic poly(vinyllactam)s are chosen from vinylpyrrolidone/dimethylaminopropylmethacrylamide/dodecyldimethylmethacrylamidopropylammonium tosylate terpolymers, vinylpyrrolidone/dimethylaminopropylmethacrylamide/cocoyldimethylmethacrylamidopropylammonium tosylate terpolymers, and vinylpyrrolidone/dimethylaminopropylmethacrylamide/lauryldimethylmethacrylamidopropyl ammonium tosylate or chloride terpolymers.

12. Composition according to any one of the preceding claims, **characterized in that** said cationic poly(vinyllactam) polymer is a vinylpyrrolidone/dimethylaminopropylmethacrylamide/lauryldimethylmethacrylamidopropylammonium chloride terpolymer.

13. Composition according to any one of the preceding claims, **characterized in that** the weight-average molecular mass of the cationic poly(vinyllactam)s is between 500 and 20 000 000, preferably between 200 000 and 2 000 000, and more preferably between 400 000 and 800 000.

14. Cosmetic composition according to any one of the preceding claims, **characterized in that** the cationic poly(vinyllactam) (s) are used in an amount ranging from 0.05 to 30% by weight, preferably in an amount ranging from 0.1 to 15% by weight and even more preferably in an amount ranging from 0.2 to 10% by weight relative to the total weight of the composition.

15. Cosmetic composition according to any one of the preceding claims, **characterized in that** said crosslinked cationic polymer is a crosslinked polymer of methacryloyloxyethyltrimethylammonium chloride of homopolymer type or of copolymer type with acrylamide.

16. Cosmetic composition according to Claim 15, **characterized in that** the acrylamide/methacryloyloxyethyltrimethylammonium chloride copolymer is obtained by copolymerizing acrylamide and dimethylaminoethyl methacrylate quaternized by methyl chloride, and **in that** the homopolymer is obtained by homopolymerizing dimethylaminoethyl methacrylate quaternized by methyl chloride, the polymerization being followed by crosslinking with a compound with olefinic unsaturation, in particular methylenebisacrylamide.

17. Cosmetic composition according to any one of Claims 1 to 16, **characterized in that** said crosslinked cationic polymer is a (20/80 by weight) acrylamide/methacryloyloxyethyltrimethylammonium chloride crosslinked copolymer in the form of a dispersion containing 50% by weight of said copolymer in mineral oil.

18. Cosmetic composition according to any one of Claims 1 to 16, **characterized in that** said crosslinked cationic polymer is a crosslinked homopolymer of methacryloyloxyethyltrimethylammonium chloride in the form of a dispersion containing 50% by weight of said homopolymer in mineral oil.

19. Cosmetic composition according to any one of the preceding claims, **characterized in that** the crosslinked cationic polymer(s) chosen from homopolymers or copolymers of methacryloyloxy (C₁-C₄) alkyltri (C₁-C₄)alkylammonium chloride is (are) present in an amount ranging from 0.01 to 30% by weight, preferably in an amount ranging from 0.05 to 20% by weight, and even more preferably in an amount ranging from 0.1 to 10% by weight relative to the total weight of the cosmetic composition.

20. Cosmetic composition according to any one of the preceding claims, **characterized in that** the nonvolatile silicone is chosen from polyorganosiloxanes which are insoluble in the composition.

21. Cosmetic composition according to Claim 20, **characterized in that** the polyorganosiloxanes are nonvolatile polyorganosiloxanes chosen from polyalkylsiloxanes, polyarylsiloxanes, polyalkylarylsiloxanes, silicone gums and resins, polyorganosiloxanes modified by organofunctional groups, and mixtures thereof.

22. Cosmetic composition according to Claim 21, **characterized in that**:
(a) the polyalkylsiloxanes are chosen from:
- polydimethylsiloxanes with trimethylsilyl end groups;
- polydimethylsiloxanes with dimethylsilanol end groups;
- poly(C₁-C₂₀)alkylsiloxanes;
(b) the polyalkylarylsiloxanes are chosen from:
- polydimethylmethylphenylsiloxanes and linear and/or branched polydimethyldiphenylsiloxanes having a viscosity of from 1 × 10⁻⁵ to 5 × 10⁻² m²/s at 25°C;
(c) the silicone gums are chosen from polydiorganosiloxanes having number-average molecular masses of between 200 000 and 1 000 000 used alone or in the form of a mixture in a solvent;
(d) the resins are chosen from resins constituted of units:
R₃SiO_{1/2}, R₂SiO_{2/2}, RSiO_{3/2}, SiO_{4/2}
in which R represents a hydrocarbon-based group having from 1 to 16 carbon atoms, or a phenyl group;
(e) the organomodified silicones are chosen from silicones comprising, in their structure, one or more organofunctional groups attached by means of a hydrocarbon-based radical.

23. Cosmetic composition according to Claim 22, **characterized in that** the silicone gums used alone or in the form of a mixture are chosen from the following structures:
- polydimethylsiloxane,
- polydimethylsiloxane/methylvinylsiloxane gums,
- polydimethylsiloxane/diphenylmethylsiloxane,
- polydimethylsiloxane/phenylmethylsiloxane,
- polydimethylsiloxane/diphenylsiloxane/methylvinylsiloxane and the following mixtures:
- mixtures formed from a polydimethylsiloxane hydroxylated at the chain end and from a cyclic polydimethylsiloxane;
- mixtures formed from a polydimethylsiloxane gum and from a cyclic silicone;
- mixtures of polydimethylsiloxanes having different viscosities.

24. Cosmetic composition according to Claim 22, **characterized in that** the organomodified silicones are chosen from polyorganosiloxanes comprising:
- polyoxyethylene and/or polyoxypropylene groups;
- substituted or unsubstituted amino groups;
- thiol groups;
- alkoxylated groups;
- hydroxyalkyl groups;
- acyloxyalkyl groups;
- alkylcarboxylic groups;
- 2-hydroxyalkylsulfonate groups;
- 2-hydroxyalkylthiosulfonate groups;
- hydroxyacylamino groups.

25. Cosmetic composition according to any one of Claims 21 to 24, **characterized in that** the polyorganosiloxanes are chosen from polyalkylsiloxanes with trimethylsilyl end groups, polyalkylsiloxanes with dimethylsilanol end groups, polyalkylarylsiloxanes, mixtures of two polydimethylsiloxanes constituted of a gum and of an oil of different viscosities, mixtures of organosiloxanes and of cyclic silicones, and organopolysiloxane resins.

26. Cosmetic composition according to any one of the preceding claims, **characterized in that** the nonvolatile silicone is present in an amount ranging from 0.05 to 10% by weight, and even more preferably in an amount ranging from 0.1 to 10% by weight relative to the total weight of the cosmetic composition.

27. Cosmetic composition according to any one of the preceding claims, **characterized in that** the weight ratio of cationic poly(vinyllactam) / crosslinked cationic polymer is between 0.1 and 10.

28. Cosmetic composition according to any one of the preceding claims, **characterized in that** the weight ratio of cationic poly(vinyllactam) / nonvolatile silicone is between 0.1 and 10.

29. Cosmetic composition according to any one of the preceding claims, **characterized in that** the weight ratio of cationic crosslinked polymer / nonvolatile silicone is between 0.2 and 5.

30. Cosmetic composition according to any one of the preceding claims, **characterized in that** it also comprises at least one additional fixing polymer.

31. Cosmetic composition according to any one of the preceding claims, **characterized in that** it also comprises at least one thickening polymer.

32. Cosmetic composition according to any one of the preceding claims, **characterized in that** it also comprises at least one cosmetic additive chosen from cationic, anionic, amphoteric or nonionic surfactants, ester-type conditioning agents, antifoams, moisturizers, emollients, plasticizers, water-soluble and liposoluble silicone-based or non-silicone-based sunscreens, permanent or temporary dyes, fragrances, peptizing agents, preservatives, ceramides and pseudo-ceramides, vitamins and provitamins including panthenol, proteins, sequestrants, solubilizers, basifying agents, anticorrosion agents, fatty substances such as vegetable, animal, mineral and synthetic oils, fatty alcohols, reducing agents or antioxidants, and oxdizing agents.

33. Cosmetic composition according to Claim 32, **characterized in that** the cosmetic additive(s) are present at a concentration ranging from 0.001 to 50% by weight relative to the total weight of the composition.

34. Cosmetic composition according to any one of the preceding claims, **characterized in that** the cosmetically acceptable medium is an aqueous, alcoholic or aqueous-alcoholic medium.

35. Cosmetic composition according to Claim 34, **characterized in that** the aqueous-alcoholic medium comprises lower C₁-C₄ alcohols, polyols, polyol monoethers and mixtures thereof.

36. Cosmetic composition according to Claim 35, **characterized in that** the alcohol is ethanol.

37. Cosmetic composition according to any one of the preceding claims, **characterized in that** it is packaged in an atomizer, a pump-action bottle or an aerosol device.

38. Cosmetic composition according to Claim 37, **characterized in that** it is packaged in an aerosol device.

39. Cosmetic composition according to Claim 38, **characterized in that** it comprises a propellant chosen from air, nitrogen, carbon dioxide, dimethyl ether, C₃ to C₅ alkanes, 1,1-difluoroethane and mixtures thereof.

40. Aerosol device formed from a container comprising a composition according to any one of the preceding claims and also a means for dispensing the composition.

41. Cosmetic treatment method, **characterized in that** it comprises the application, in particular to the hair, of a cosmetic composition according to any one of Claims 1 to 39.

42. Cosmetic treatment method according to Claim 41, **characterized in that** the application of said composition is not followed by a rinsing operation.

43. Use of a cosmetic composition according to any one of Claims 1 to 39 for hair shaping and/or form retention of the hairstyle.
